# EUROPEAN PATENT APPLICATION

(11) **EP 4 644 549 A1**
(43) Date of publication of application: **05.11.2025**
(21) Application number: 23911934.0
(22) Date of filing: 21.12.2023
(51) Int. Cl.: C12N 15/31, A61K 38/16, A61P 31/04, C07K 1/00, C07K 14/335, C12N 1/00, C12N 1/15, C12N 1/19, C12N 1/21, C12N 5/10, C12N 15/63, C12P 21/02

(54) **ANTIMICROBIAL PEPTIDE AND METHOD FOR PRODUCING SAME**

(30) Priority: 27.12.2022 JP 2022210404
(71) Applicant: Sumitomo Chemical Company, Limited, Tokyo 103-6020 (JP)
(72) Inventor: KUWAHARA, Hiroshi, Osaka-shi, Osaka 554-8558 (JP); OZAWA, Naoya, Osaka-shi, Osaka 554-8558 (JP); MIKATA, Kazuki, Osaka-shi, Osaka 554-8558 (JP); AOKI, Mikio, Osaka-shi, Osaka 554-8558 (JP)
(74) Representative: Vossius & Partner Patentanwälte Rechtsanwälte mbB
(86) International application number: PCT/JP2023/045944
(87) International publication number: WO 2024/143154

(57) **Abstract**

Provided is a polypeptide with antimicrobial activity comprising an amino acid sequence with a sequence identity of 85% to 100% to an amino acid sequence as set forth in any one of SEQ ID NO: 1 to SEQ ID NO: 6 or an amino acid sequence as set forth in any one of SEQ ID NO: 1 to SEQ ID NO: 6 with deletion, substitution, insertion, and/or addition of one or several amino acid residues.

## Description

### TECHNICAL FIELD

The present invention relates to an antimicrobial peptide and a method of producing the same.

### BACKGROUND ART

Antimicrobial peptides are produced by a wide range of living things including bacteria (such as lactic acid bacteria, for example), animals (such as insects and mammals, for example), and plants, and play an important defense role for living things. Since they act quickly as compared to antibiotics and do not remain in the environment due to degradation into amino acids by digestive enzymes and the like, antimicrobial peptides are less likely to give rise to resistant bacteria. Antimicrobial peptides are expected to be useful as a means to deal with drug-resistant bacteria, which is a serious social issue to address today.

### CITATION LIST

### PATENT LITERATURE

PTL 1: U.S. Patent No. 4584199
PTL 2: U.S. Patent No. 3295989
PTL 3: International Patent Laying-Open No. WO 89/12399
PTL 4: International Patent Laying-Open No. WO 2004/029082

### SUMMARY OF INVENTION

### TECHNICAL PROBLEM

As an antimicrobial peptide produced by lactic acid bacteria (Lactococcus lactis A TCC-11454 strain), nisin A is known. Nisin A exhibits high antimicrobial activity against many gram-positive bacteria including heat-resistant spore-forming bacteria, is stable against acid, and is degraded by intestinal digestive enzymes into amino acids. Therefore, nisin A has been used as a food preservative in practical settings in at least fifty countries. However, nisin A is unstable within the neutral range and the alkaline range, and not highly thermostable within the neutral range. For these reasons, nisin A is only used as preservatives for food and the like which are often sold with their pH adjusted to the acidic range. Moreover, since nisin A contains abnormal amino acids (such as lanthionine, for example) resulting from post-translational modification in cells and also has a crosslink structure based on monosulfide bonding of lanthionine and/or the like, it is difficult to synthesize it chemically or by recombinant DNA techniques or by in vitro translation. In addition, it is reported that nisin A is not effective against gram-negative bacteria (such as Escherichia coli, for example) and fungi (such as candida, for example). U.S. Patent No. 4584199 specification (PTL 1), U.S. Patent No. 3295989 specification (PTL 2), International Patent Laying-Open No. WO 89/12399 (PTL 3), and International Patent Laying-Open No. WO 2004/029082 (PTL 4) disclose use of nisin for food preservation.

Under such circumstances, an antimicrobial peptide that is highly stable within the neutral range and the alkaline range and also highly thermostable is demanded. The present invention has been devised in light of the above-mentioned circumstances, and aims at providing a polypeptide with antimicrobial activity that is highly stable within the neutral range and the alkaline range and also highly thermostable, as well as a method of producing the same.

### SOLUTION TO PROBLEM

The present invention relates to the following items.
[1] A polypeptide with antimicrobial activity, comprising:
   an amino acid sequence with a sequence identity of 85% to 100% to an amino acid sequence as set forth in any one of SEQ ID NO: 1 to SEQ ID NO: 6; or
   an amino acid sequence as set forth in any one of SEQ ID NO: 1 to SEQ ID NO: 6 with deletion, substitution, insertion, and/or addition of one or several amino acid residues.
[2] A nucleic acid comprising a base sequence encoding the polypeptide with antimicrobial activity according to [1].
[3] The nucleic acid according to [2], comprising a base sequence as set forth in any one of SEQ ID NO: 7 to SEQ ID NO: 12
[4] A vector comprising the nucleic acid according to [2] or [3].
[5] An in-vitro translation template molecule including the nucleic acid according to [2] or [3].
[6] The in-vitro translation template molecule according to [5], wherein the in-vitro translation template molecule is an RNA.
[7] A transformant comprising the nucleic acid according to [2] or [3] or the vector according to [4].
[8] A method of producing a polypeptide with antimicrobial activity, the method comprising one of:
   (A) synthesizing the polypeptide with antimicrobial activity according to [1] by a chemical synthesis method;
   (B) translating the polypeptide with antimicrobial activity from the in-vitro translation template molecule according to [5] or [6];
   (C) culturing the transformant according to [7]; and
   (D) culturing at least one type of lactic acid bacteria selected from the group consisting of accession numbers NITE BP-03198, NITE BP-03200, and NITE BP-03201.
[9] A combination of polypeptides with antimicrobial activity, comprising:
   a combination of a first polypeptide and a second polypeptide; or
   a combination of a third polypeptide and a fourth polypeptide, wherein
   the first polypeptide includes an amino acid sequence with a sequence identity of 85% to 100% to an amino acid sequence as set forth in SEQ ID NO: 3, or the amino acid sequence as set forth in SEQ ID NO: 3 with deletion, substitution, insertion, and/or addition of one or several amino acid residues,
   the second polypeptide includes an amino acid sequence with a sequence identity of 85% to 100% to an amino acid sequence as set forth in SEQ ID NO: 4, or the amino acid sequence as set forth in SEQ ID NO: 4 with deletion, substitution, insertion, and/or addition of one or several amino acid residues,
   the third polypeptide includes an amino acid sequence with a sequence identity of 85% to 100% to an amino acid sequence as set forth in SEQ ID NO: 13, or the amino acid sequence as set forth in SEQ ID NO: 13 with deletion, substitution, insertion, and/or addition of one or several amino acid residues, and
   the fourth polypeptide includes an amino acid sequence with a sequence identity of 85% to 100% to an amino acid sequence as set forth in SEQ ID NO: 14, or the amino acid sequence as set forth in SEQ ID NO: 14 with deletion, substitution, insertion, and/or addition of one or several amino acid residues.
[10] A combination of nucleic acids including base sequences respectively encoding individual polypeptides of the combination of polypeptides with antimicrobial activity according to [9], wherein
   the combination of nucleic acids includes a combination of a first nucleic acid and a second nucleic acid or a combination of a third nucleic acid and a fourth nucleic acid,
   the first nucleic acid includes a base sequence encoding the first polypeptide,
   the second nucleic acid includes a base sequence encoding the second polypeptide,
   the third nucleic acid includes a base sequence encoding the third polypeptide, and
   the fourth nucleic acid includes a base sequence encoding the fourth polypeptide.
[11] The combination of nucleic acids according to [10], wherein
   the first nucleic acid includes a base sequence as set forth in SEQ ID NO: 9,
   the second nucleic acid includes a base sequence as set forth in SEQ ID NO: 10,
   the third nucleic acid includes a base sequence as set forth in SEQ ID NO: 15, and
   the fourth nucleic acid includes a base sequence as set forth in SEQ ID NO: 16.
[12] A vector comprising the combination of nucleic acids according to [10] or [11].
[13] An in-vitro translation template molecule including the combination of nucleic acids according to [10] or [11].
[14] The in-vitro translation template molecule according to [13], wherein the in-vitro translation template molecule is an RNA.
[15] A transformant comprising the combination of nucleic acids according to [10] or [11] or the vector according to [12].
[16] A method of producing a combination of polypeptides with antimicrobial activity, the method comprising one of:
   (A) synthesizing individual polypeptides of the combination of polypeptides according to [9] by a chemical synthesis method;
   (B) translating the individual polypeptides of the combination of polypeptides from the in-vitro translation template molecule according to [13] or [14];
   (C) culturing the transformant according to [15]; and
   (D) culturing lactic acid bacteria under an accession number of NITE BP-03200.
[17] An antimicrobial composition comprising:
   the polypeptide with antimicrobial activity according to [1] or the combination of polypeptides with antimicrobial activity according to [9]; and
   an additive.

### ADVANTAGEOUS EFFECTS OF INVENTION

The present invention makes it possible to provide a polypeptide with antimicrobial activity that is highly stable within the neutral range and the alkaline range and also highly thermostable, as well as a method of producing the same.

### BRIEF DESCRIPTION OF DRAWINGS

Fig. 1 is a schematic view illustrating an antimicrobial activity evaluation test.
Fig. 2 is a photograph showing results of an antimicrobial activity evaluation test performed on a polypeptide of Examples.

### DESCRIPTION OF EMBODIMENTS

In the following, a detailed description will be given of an embodiment of the present invention (hereinafter also referred to as "the present embodiment"). It should be noted that the scope of the present invention is not limited to the below embodiment. In the present specification, the expression "from A to Z" or "A to Z" refers to the upper limit and the lower limit of a range (that is, it means not less than A and not more than Z), and if a description of unit is attached only to Z but not to A, A has the same unit as that of Z. In the present specification, the expression "include" encompasses the concepts of "consist of" and "consist solely of".

### [Polypeptide with Antimicrobial Activity]

A polypeptide with antimicrobial activity according to the present embodiment is a polypeptide with antimicrobial activity comprising:
an amino acid sequence with a sequence identity of 85% to 100% to an amino acid sequence as set forth in any one of SEQ ID NO: 1 to SEQ ID NO: 6; or
an amino acid sequence as set forth in any one of SEQ ID NO: 1 to SEQ ID NO: 6 with deletion, substitution, insertion, and/or addition of one or several amino acid residues.

Hereinafter, a polypeptide with antimicrobial activity may be simply expressed as "a polypeptide".

In the present embodiment, "antimicrobial activity" means a characteristic to inhibit growth of target microorganisms. The antimicrobial activity can be evaluated by an antimicrobial activity evaluation test that is described below in the Examples section.

The target microorganisms may be pathogenic or hazardous. The target microorganisms may be gram-positive bacteria, or may be gram-negative bacteria, or may be fungi. Examples of the gram-positive bacteria include Lactobacillus sakei, Streptococcus uberis, Staphylococcus aureus, and the like; examples of the gram-negative bacteria include Escherichia coli and the like; and examples of the fungi include Candida albicans and the like.

The polypeptide according to the present embodiment may include an amino acid sequence with a sequence identity of 85% to 100%, for example, to an amino acid sequence as set forth in any one of SEQ ID NO: 1 to SEQ ID NO: 6. In an aspect of the present embodiment, the lower limit to the sequence identity may be 90% or more, or may be 95% or more, or may be 96% or more, or may be 97% or more, or may be 98% or more, or may be 99% or more. The polypeptide according to the present embodiment may consist solely of an amino acid sequence as set forth in any one of SEQ ID NO: 1 to SEQ ID NO: 6.

In the present embodiment, "sequence identity" means the following: two amino acid sequences are aligned by a mathematical algorithm known in the technical field (preferably, this algorithm tolerates gap introduction to one or both of the sequences for the sake of optimum alignment), and, in the resulting optimum alignment, the proportion (%) of matching amino acids across the entire overlapping amino acid sequences is referred to as "sequence identity". A person skilled in the art can easily check "sequence identity" between amino acid sequences. For example, NCBI BLAST (National Center for Biotechnology Information Basic Local Alignment Search Tool) can be used. This method can also be used for determining sequence identity between base sequences.

The polypeptide according to the present embodiment may include an amino acid sequence as set forth in any one of SEQ ID NO: 1 to SEQ ID NO: 6 with deletion, substitution, insertion, and/or addition of one or several amino acid residues.

"One or several" varies depending on the position, type, and the like of the amino acid residue(s) in the conformation of the protein, and, for example, it may be from 1 to 10. The polypeptide according to the present embodiment may be a polypeptide that includes an amino acid sequence as set forth in any one of SEQ ID NO: 1 to SEQ ID NO: 6 with deletion, substitution, insertion, and/or addition of 1 to 10 amino acid residues, for example. The upper limit to the number of amino acid residues thus deleted, substituted, inserted, and/or added may be 9 or less, or may be 8 or less, or may be 7 or less, or may be 6 or less, or may be 5 or less, or may be 4 or less, or may be 3 or less, or may be 2 or less.

In an aspect of the present embodiment, examples of "an amino acid sequence with deletion, substitution, insertion, and/or addition of one or several amino acid residues" include amino acid sequences that are obtained as a result of deletion, substitution, insertion, and/or addition and that have a sequence identity of 80% or more, or 85% or more, or 90% or more, or 95% or more, or 97% or more, or 98% or more, or 99% or more to the original amino acid sequence.

An example of the substitution, deletion, insertion, and/or addition of amino acid residues is a conservative variation that properly retains the function of the polypeptide. A typical conservative variation is conservative substitution. Examples of conservative substitution include the following variations. In the case where an aromatic amino acid is at the moiety of substitution, conservative substitution refers to a variation in which substitution takes place between Phe, Trp, and Tyr, and in the case where a hydrophobic amino acid is at the moiety of substitution, conservative substitution refers to a variation in which substitution takes place between Leu, Ile, and Val. In the case of a polar amino acid, conservative substitution refers to a variation in which substitution takes place between Gln and Asn, and in the case of a basic amino acid, conservative substitution refers to a variation in which substitution takes place between Lys, Arg, and His. In the case of an acidic amino acid, conservative substitution refers to a variation in which substitution takes place between Asp and Glu, and in the case of a hydroxy-containing amino acid, conservative substitution refers to a variation in which substitution takes place between Ser and Thr. Examples of the substitution that is regarded as conservative substitution include substitution from Ala to Ser or Thr, substitution from Arg to Gln, His, or Lys, substitution from Asn to Glu, Gln, Lys, His, or Asp, substitution from Asp to Asn, Glu, or Gln, substitution from Cys to Ser or Ala, substitution from Gln to Asn, Glu, Lys, His, Asp, or Arg, substitution from Glu to Gly, Asn, Gln, Lys, or Asp, substitution from Gly to Pro, substitution from His to Asn, Lys, Gln, Arg, or Tyr, substitution from Ile to Leu, Met, Val, or Phe, substitution from Leu to Ile, Met, Val, or Phe, substitution from Lys to Asn, Glu, Gln, His, or Arg, substitution from Met to Ile, Leu, Val, or Phe, substitution from Phe to Trp, Tyr, Met, Ile, or Leu, substitution from Ser to Thr or Ala, substitution from Thr to Ser or Ala, substitution from Trp to Phe or Tyr, substitution from Tyr to His, Phe, or Trp, and substitution from Val to Met, Ile, or Leu.

Each of the amino acid sequences as set forth in SEQ ID NOs: 1 and 2 is the amino acid sequence of a mature polypeptide predicted from the genome sequence of lactic acid bacteria named Lactiplantibacillus plantarum under NITE BP-03198.

Each of the amino acid sequences as set forth in SEQ ID NOs: 3 and 4 and the amino acid sequence as set forth in SEQ ID NO: 5 (X in the amino acid sequence is Ala) is the amino acid sequence of a mature polypeptide predicted from the genome sequence of lactic acid bacteria named Lactiplantibacillus plantarum under NITE BP-03200.

Each of the amino acid sequence as set forth in SEQ ID NO: 5 (X in the amino acid sequence is Val) and the amino acid sequence as set forth in SEQ ID NO: 6 is the amino acid sequence of a mature polypeptide predicted from the genome sequence of lactic acid bacteria named Lactiplantibacillus plantarum under NITE BP-03201.

### [Combination of Polypeptides with Antimicrobial Activity]

A combination of polypeptides with antimicrobial activity according to the present embodiment is
a combination of polypeptides with antimicrobial activity, comprising:
a combination of a first polypeptide and a second polypeptide; or
a combination of a third polypeptide and a fourth polypeptide, wherein
the first polypeptide includes an amino acid sequence with a sequence identity of 85% to 100% to an amino acid sequence as set forth in SEQ ID NO: 3, or the amino acid sequence as set forth in SEQ ID NO: 3 with deletion, substitution, insertion, and/or addition of one or several amino acid residues,
the second polypeptide includes an amino acid sequence with a sequence identity of 85% to 100% to an amino acid sequence as set forth in SEQ ID NO: 4, or the amino acid sequence as set forth in SEQ ID NO: 4 with deletion, substitution, insertion, and/or addition of one or several amino acid residues,
the third polypeptide includes an amino acid sequence with a sequence identity of 85% to 100% to an amino acid sequence as set forth in SEQ ID NO: 13, or the amino acid sequence as set forth in SEQ ID NO: 13 with deletion, substitution, insertion, and/or addition of one or several amino acid residues, and
the fourth polypeptide includes an amino acid sequence with a sequence identity of 85% to 100% to an amino acid sequence as set forth in SEQ ID NO: 14, or the amino acid sequence as set forth in SEQ ID NO: 14 with deletion, substitution, insertion, and/or addition of one or several amino acid residues.

Herein, "a combination of polypeptides" may be in the form of a mixture of two types of polypeptides of interest, or may be in the form of two types of polypeptides of interest that are present independently of one another but to be used together, or may be in the form of a single molecule composed of two types of polypeptides of interest that are bound to one another in tandem via a linker peptide (such as 6×Gly, for example) or a linker compound.

Each of the amino acid sequences as set forth in SEQ ID NOs: 13 and 14 is the amino acid sequence of a mature polypeptide predicted from the genome sequence of lactic acid bacteria named Lactiplantibacillus plantarum under NITE BP-03200.

The polypeptide according to the present embodiment has antimicrobial activity against a wide range of microbial species, and, for example, it has antimicrobial activity against gram-positive bacteria, gram-negative bacteria, fungi, and the like. Antimicrobial activity of the polypeptide can be measured by an antimicrobial activity test method described below (see Experiment 3), for example. The polypeptide according to the present embodiment is highly pH-stable, and has antimicrobial activity not only within the acidic range (pH4.0, for example) but also within the neutral range (pH7.4, for example) and the alkaline range (pH10.0, for example). The polypeptide according to the present embodiment is highly thermostable, and, for example, has antimicrobial activity after heat treatment (at temperatures of 80°C, 100°C, 121°C, and the like) within the neutral range. In this way, the polypeptide according to the present embodiment is a naturally-occurring polypeptide with high antimicrobial activity, and due to its wide applicable pH range and its stability against heat and the like, it is widely applicable as an antimicrobial component.

The polypeptide according to the present embodiment may include a signal peptide (a leader peptide), and may be a precursor polypeptide for the above-mentioned polypeptide. The precursor polypeptide can be cleaved by peptidase either inside or outside of cells to become the above-mentioned polypeptide (a mature polypeptide). Usually, the precursor polypeptide includes an amino acid sequence that is to be cleaved by a particular peptidase. Precursor polypeptides for the polypeptides formed of the amino acid sequences as set forth in SEQ ID NOs: 1 to 6 may be polypeptides formed of the amino acid sequences as set forth in SEQ ID NOs: 25 to 30, respectively (Table 1). Precursor polypeptides for the polypeptides formed of the amino acid sequences as set forth in SEQ ID NOs: 13 and 14 may be polypeptides formed of the amino acid sequences as set forth in SEQ ID NOs: 31 and 32 (Table 1). The polypeptide according to the present embodiment may be a secretory polypeptide, or may be a membrane-bound polypeptide.

In another aspect of the present embodiment, the polypeptide may further include a peptide that serves as a purification tag. Examples of the purification tag include a polyhistidine sequence (6×His) and the like.

### [Antimicrobial Composition]

An antimicrobial composition according to the present embodiment is
an antimicrobial composition comprising:
the above-mentioned polypeptide with antimicrobial activity or the above-mentioned combination of polypeptides with antimicrobial activity; and
an additive.

The antimicrobial composition may be either a growth inhibitor agent or a bactericide to be used against microorganisms such as bacteria. Examples of the additive included in the antimicrobial composition include a surfactant, a preserving agent, a preservative, a pH regulator, a thickener, an excipient, and a fragrance. One type of the additives may be included alone, or two or more types may be included in combination.

The surfactant may be an anionic surfactant, a nonionic surfactant, or an amphoteric surfactant. Examples of the anionic surfactant include N-acylamino acid salts, α-olefin sulfonic acid salts, N-acylsulfonic acid salts, alkyl sulfuric acid salts (such as sodium lauryl sulfate, for example), glycerol fatty acid ester sulfuric acid salts, and the like. Examples of the nonionic surfactant include polyoxyethylene alkyl ether, polyoxyethylene-polyoxypropylene block copolymer, polyoxyethylene hydrogenated castor oil, glyceryl ester polyoxyethylene ether, alkylol amide, sucrose fatty acid ester, glycerol fatty acid ester, sorbitan fatty acid ester, propylene glycol fatty acid ester, glycerol organic acid fatty acid ester, polyglycerol fatty acid ester, calcium stearoyl lactylate, sodium stearoyl lactylate, polyoxyethylene sorbitan fatty acid ester, alkyl glycoside, and the like. Examples of the amphoteric surfactant include alkyl betaine-based surfactants, amine oxide-based surfactants, imidazolinium betaine-based surfactants. Specific examples thereof include 2-alkyl-N-carboxymethyl-N-hydroxyethyl imidazolinium betaine, lauryl dimethylaminoacetic acid betaine, coconut alkyl betaine (coconut alkyl dimethylaminoacetic acid betaine), stearyldimethylaminoacetic acid betaine, sodium stearyl dimethyl betaine, cocamide alkyl betaine, palm acid amidopropyl betaine, lauryl amidopropyl betaine, ricinoleic acid amidopropyl betaine, stearyl dihydroxyethyl betaine, and the like.

Examples of the preserving agent or the preservative include sodium benzoate, p-hydroxybenzoic acid esters such as methylparaben, ethylparaben, butylparaben, isopropylparaben, propylparaben, isobutylparaben, and benzylparaben, alcohols such as phenoxyethanol and ethanol, sorbic acid, benzoic acid, dehydroacetic acid, propionic acid, and salts thereof, salts such as sodium chloride, ethylenediaminetetraacetate, benzalkonium chloride, benzethonium chloride, cetylpyridinium chloride, alkyl diaminoethyl glycine hydrochloride, and the like.

Examples of the pH regulator include acids and alkalis such as acetic acid, hydrochloric acid, sulfuric acid, nitric acid, citric acid, phosphoric acid, malic acid, gluconic acid, maleic acid, succinic acid, glutamic acid, pyrophosphoric acid, tartaric acid, acetic acid sodium hydroxide, potassium hydroxide, sodium hydroxide, sodium acetate, sodium carbonate, potassium carbonate, sodium citrate, sodium hydrogen citrate, phosphoric acid, sodium phosphate, sodium monohydrogen phosphate, sodium dihydrogen phosphate, and potassium dihydrogen phosphate, buffers, and the like.

Examples of the thickener include methylcellulose, hydroxyethylcellulose, hydroxypropylcellulose, carboxymethylcellulose, and salts thereof, organic bonding agents such as pullulan, gelatin, carrageenan, sodium alginate, xanthan gum, sodium polyacrylate, gum arabic, guar gum, locust bean gum, polyvinyl alcohol, polyvinylpyrrolidone, and carboxyvinyl polymer, thickening silicic anhydride, inorganic bonding agents such as bentonite, and the like.

Examples of the excipient include crystalline cellulose, powder cellulose, potato starch, corn starch, light silicic anhydride, hydrous silicon dioxide, silicon dioxide, precipitated calcium carbonate, anhydrous calcium hydrogen phosphate, magnesium oxide, calcium lactate, calcium silicate, magnesium aluminometasilicate, synthesized hydrotalcite, synthesized aluminum silicate, lactose, white sugar, D-mannitol, erythritol, glucose, fructose, talc, dextrin, cyclodextrin, and the like.

Examples of the fragrance include peppermint, Aloe vera sap, and the like.

The polypeptide with antimicrobial activity and the antimicrobial composition according to the present embodiment are used for inhibiting growth of microorganisms such as bacteria, or for killing such microorganisms.

### [Nucleic Acid Encoding Polypeptide with Antimicrobial Activity]

A nucleic acid according to the present embodiment is a nucleic acid that includes a base sequence encoding the above-mentioned polypeptide with antimicrobial activity. The nucleic acid may be a DNA or an RNA. The nucleic acid may or may not include a start codon on the 5' end side and a stop codon on the 3' end side. The nucleic acid may or may not include an intron sequence. An example of the nucleic acid according to the present embodiment includes a base sequence as set forth in any one of SEQ ID NO: 7 to SEQ ID NO: 12. The base sequences as set forth in SEQ ID NOs: 7 to 12 encode amino acids of SEQ ID NO: 1 to SEQ ID NO: 6, respectively. It should be noted that when the nucleic acid is an RNA, the base sequence of the RNA is the base sequence of the corresponding DNA with thymine replaced by uracil.

In an aspect of the present embodiment, the base sequences as set forth in SEQ ID NOs: 17 and 18 are sequences derived from the genome sequence of lactic acid bacteria named Lactiplantibacillus plantarum (NITE BP-03198), and encode precursor polypeptides formed of the amino acid sequences as set forth in SEQ ID NOs: 25 and 26, respectively. The base sequences as set forth in SEQ ID NOs: 7 and 8 encode polypeptides formed of the amino acid sequences as set forth in SEQ ID NOs: 1 and 2, respectively.

The base sequences as set forth in SEQ ID NOs: 19 to 21 are sequences derived from the genome sequence of lactic acid bacteria named Lactiplantibacillus plantarum (NITE BP-03200), and encode precursor polypeptides formed of the amino acid sequences as set forth in SEQ ID NOs: 27 to 29, respectively. The base sequences as set forth in SEQ ID NOs: 9 to 11 encode polypeptides formed of the amino acid sequences as set forth in SEQ ID NOs: 3 to 5, respectively. As for NITE BP-03200, the base denoted as y in the base sequences as set forth in SEQ ID NOs: 11 and 21 is cytosine, and the amino acid residue denoted as X in the amino acid sequences as set forth in SEQ ID NOs: 5 and 29 is alanine.

The base sequences as set forth in SEQ ID NOs: 21 and 22 are sequences derived from the genome sequence of lactic acid bacteria named Lactiplantibacillus plantarum (NITE BP-03201), and encode precursor polypeptides formed of the amino acid sequences as set forth in SEQ ID NOs: 29 and 30, respectively. The base sequences as set forth in SEQ ID NOs: 11 and 12 encode polypeptides formed of the amino acid sequences as set forth in SEQ ID NOs: 5 and 6, respectively. As for NITE BP-03201, the base denoted as y in the base sequences as set forth in SEQ ID NOs: 11 and 21 is thymine, and the amino acid residue denoted as X in the amino acid sequences as set forth in SEQ ID NOs: 5 and 29 is valine.

The nucleic acid is not limited to the base sequences of the above-mentioned lactic acid bacteria under NITE BP-03198, NITE BP-03200, and NITE BP-03201, and may be a nucleic acid that includes a base sequence in which a codon encoding an amino acid in a coding region is substituted by another codon (equivalent codon) encoding the same amino acid. The nucleic acid according to the present embodiment may be a nucleic acid that includes a base sequence in which the codon usage is changed for the purpose of enhancing expression of the polypeptide according to the present embodiment.

In an aspect of the present embodiment, the nucleic acid may further include a base sequence encoding a peptide that serves as a purification tag.

The nucleic acid according to the present embodiment can be obtained by chemical synthesis, or by PCR using the genome sequence of lactic acid bacteria under NITE BP-03198, NITE BP-03200, and NITE BP-03201 as a template, or by other methods.

### [Combination of Nucleic Acids Encoding Combination of Polypeptides with Antimicrobial Activity]

A combination of nucleic acids according to the present embodiment is a combination of nucleic acids including base sequences respectively encoding individual polypeptides of the above-mentioned combination of polypeptides with antimicrobial activity, wherein
the combination of nucleic acids includes a combination of a first nucleic acid and a second nucleic acid or a combination of a third nucleic acid and a fourth nucleic acid,
the first nucleic acid includes a base sequence encoding the first polypeptide,
the second nucleic acid includes a base sequence encoding the second polypeptide,
the third nucleic acid includes a base sequence encoding the third polypeptide, and
the fourth nucleic acid includes a base sequence encoding the fourth polypeptide.

Herein, "a combination of nucleic acids" may be in the form of a mixture of two types of nucleic acids of interest, or may be in the form of two types of nucleic acids of interest that are present independently of one another but to be used together, or may be in the form of a single molecule composed of two types of nucleic acids of interest that are bound to one another in tandem via a nucleic acid encoding a linker peptide (such as 6×Gly, for example).

In an aspect of the present embodiment, preferably, the first nucleic acid includes the base sequence as set forth in SEQ ID NO: 9, the second nucleic acid includes the base sequence as set forth in SEQ ID NO: 10, the third nucleic acid includes the base sequence as set forth in SEQ ID NO: 15, and the fourth nucleic acid includes the base sequence as set forth in SEQ ID NO: 16.

The base sequences as set forth in SEQ ID NOS: 23 and 24 are sequences derived from the genome sequence of lactic acid bacteria named Lactiplantibacillus plantarum (NITE BP-03200), and encode precursor polypeptides formed of the amino acid sequences as set forth in SEQ ID NOs: 31 and 32, respectively. The base sequences as set forth in SEQ ID NOS: 15 and 16 encode polypeptides formed of the amino acid sequences as set forth in SEQ ID NOS: 13 and 14, respectively.

Each nucleic acid in the above-mentioned combination of nucleic acids may be a DNA or an RNA. The nucleic acid may or may not include a start codon on the 5' end side, and a stop codon on the 3' end side. The nucleic acid may or may not include an intron sequence. It should be noted that when the nucleic acid is an RNA, the base sequence of the RNA is the base sequence of the corresponding DNA with thymine replaced by uracil.

Each nucleic acid in the above-mentioned combination of nucleic acids is not limited to the base sequence of the above-mentioned lactic acid bacteria under NITE BP-03200, and may be a nucleic acid that includes a base sequence in which a codon encoding an amino acid in a coding region is substituted by another codon (equivalent codon) encoding the same amino acid. The nucleic acid may be a nucleic acid that includes a base sequence in which the codon usage is changed for the purpose of enhancing expression of the to-be-encoded polypeptide.

In an aspect of the present embodiment, each nucleic acid in the above-mentioned combination of nucleic acids may further include a base sequence encoding a peptide that serves as a purification tag or a linker peptide.

Each nucleic acid in the above-mentioned combination of nucleic acids can be obtained by chemical synthesis, or by PCR using the genome sequence of lactic acid bacteria under NITE BP-03200 as a template, or by other methods.

### [Vector]

A vector according to the present embodiment includes the above-mentioned nucleic acid or the above-mentioned combination of nucleic acids. The vector is a nucleic acid molecule that is capable of amplifying and retaining a DNA, and examples thereof include expression vectors and cloning vectors. In one example, the nucleic acid is inserted into an expression vector, introduced into a host cell and the like, and expresses a polypeptide with antimicrobial activity. The vector, after introduced in a host cell, is capable of expressing a polypeptide in the host cell. The expression vector may have a promoter sequence and a terminator sequence for expression of a gene it harbors. The above-mentioned vector can be constructed by introducing the above-mentioned nucleic acid into a basic vector with the use of an ordinary genetic engineering technique. The above-mentioned vector may have a selection marker sequence.

In an aspect of the present embodiment, when the vector includes the above-mentioned combination of nucleic acids, the vector (a single vector) may include both the two types of nucleic acids that constitute the combination. Alternatively, the vector may include a first vector and a second vector, where the first vector may include the first nucleic acid and the second vector may include the second nucleic acid. Alternatively, the vector may include a first vector and a second vector, where the first vector may include the third nucleic acid and the second vector may include the fourth nucleic acid.

For example, the vector may be a bacterial plasmid-derived vector, a yeast plasmid-derived vector, a viral vector, a cosmid vector, a phagemid vector, an artificial chromosome vector, and/or the like. Examples thereof include pBR322, pUC plasmid vector, pET-based plasmid vector, and the like. Specifically, in the case where Escherichia coli is used as the host cell, pUC19, pUC18, pUC119, pBluescriptII, pET32, and/or the like may be used. In the case where a mammalian cell is used as the host cell, pRc/RSV, pRc/CMV, a retroviral vector, an adenoviral vector, an adeno-associated virus vector, and/or the like may be used, for example.

When introduced into the vector, the above-mentioned nucleic acid or the above-mentioned combination of nucleic acids is inserted downstream of a promoter in such a manner that the promoter is capable of functioning. The promoter may be any promoter suitable for the host that is to be used for gene expression. For example, when the host is an animal cell, SRα promoter, SV40 promoter, LTR promoter, CMV (cytomegalovirus) promoter, RSV (Rous sarcoma virus) promoter, MoMuLV (Moloney murine leukemia virus) LTR, HSV-TK (herpes simplex virus thymidine kinase) promoter, and/or the like is used. Among these, CMV promoter, SRα promoter, and/or the like is preferable. When the host is Escherichia coli, T7 promoter, cspA promoter, trp promoter, lac promoter, recA promoter, λPL promoter, lpp promoter, tac promoter, and/or the like is preferable. When the host is bacteria of the genus Bacillus, SPO1 promoter, SPO2 promoter, penP promoter, and/or the like is preferable. When the host is yeast, Gal1/10 promoter, PHO5 promoter, PGK promoter, GAP promoter, ADH promoter, AOX1 promoter, and/or the like is preferable. When the host is aspergilli, ENOA promoter, TAA promoter, GLA promoter, AGL promoter, and/or the like is preferable. When the host is an insect cell, polyhedrin promoter, P10 promoter, and/or the like is preferable. When the host is a plant cell, CaMV35S promoter, CaMV19S promoter, NOS promoter, and/or the like is preferable.

As the expression vector, not only the above-mentioned ones but also those harboring an enhancer, a splicing signal, a poly (A) addition signal, a selection marker such as a drug resistance gene and/or an auxotrophic complementary gene, an origin of replication, and/or the like can be used, depending on the purpose.

### [In-Vitro Translation Template Molecule]

An in-vitro translation template molecule according to the present embodiment includes the above-mentioned nucleic acid or the above-mentioned combination of nucleic acids. Herein, the "in-vitro translation template molecule" means a nucleic acid molecule that serves as a template for translation in a cell-free protein synthesis system. The in-vitro translation template molecule may be a DNA, or may be an RNA. The in-vitro translation template molecule may be a single strand, or may be double strands. The in-vitro translation template molecule may have a promoter sequence and a terminator sequence for expression of the gene it has. Hereinafter, the in-vitro translation template molecule may also be expressed as "a translation template molecule".

In an aspect of the present embodiment, when the in-vitro translation template molecule includes the above-mentioned combination of nucleic acids, the in-vitro translation template molecule (a single in-vitro translation template molecule) may include both the two types of nucleic acids that constitute the combination. Alternatively, the in-vitro translation template molecule may include a first translation template molecule and a second translation template molecule, where the first translation template molecule may include the first nucleic acid and the second translation template molecule may include the second nucleic acid. Alternatively, the in-vitro translation template molecule may include a first translation template molecule and a second translation template molecule, where the first translation template molecule may include the third nucleic acid and the second translation template molecule may include the fourth nucleic acid.

When introduced into the in-vitro translation template molecule, the nucleic acid or the combination of nucleic acids is inserted downstream of a promoter in such a manner that the promoter is capable of functioning. Examples of the promoter include the above-mentioned promoters.

### [Transformant]

A transformant according to the present embodiment is a cell in which the above-mentioned nucleic acid or the above-mentioned vector is introduced. The transformant is capable of expressing the polypeptide according to the present embodiment.

The host cell into which the vector is to be introduced can be a eukaryotic cell or a prokaryotic cell. Specific examples thereof include bacteria, fungi, plant cells, animal cells, and insect cells. The host cell may be a cell of yeast, Escherichia coli, aspergilli, or a mammal. The mammal may be a human, a cow, a horse, sheep, a monkey and an ape, a pig, a mouse, a rat, a hamster, a guinea pig, a rabbit, a dog, and/or the like. In an aspect of the present embodiment, the host cell may be a cell of yeast (such as BG10 and BG11), Escherichia coli (such as BL21 and DH5α), HEK 293T, CHO, Expi293F, and/or ExpiCHO, for example.

Examples of the method for introducing the nucleic acid or the vector into the host cell include chemical techniques such as a calcium phosphate method, a DEAE-dextran method, and a cationic liposome method; biological techniques such as adenoviral vector, vaccinia virus vector, retroviral vector, and HVJ liposome; physical techniques such as electroporation, DNA direct injection, and gene gun; and the like. An introduction method suitable for the host cell into which the nucleic acid or the vector is to be introduced can be selected.

In the host cell, the vector may be retained outside the chromosome or inside the chromosome.

The transformant in which the nucleic acid or the vector is introduced may be selected with the use of a selection marker. For example, a selection marker gene is introduced into the host cell simultaneously with the nucleic acid according to the present embodiment, and cultured by a method suitable for the characteristics of the selection marker. In the case where the selection marker gene is a gene capable of providing drug resistance against a selective agent that has activity to kill the host cell, the host cell in which the nucleic acid is introduced can be cultured in a medium that contains the selective agent.

### [Method of Producing Polypeptide with Antimicrobial Activity]

A method of producing a polypeptide with antimicrobial activity according to the present embodiment comprises one of:
(A) synthesizing the above-mentioned polypeptide with antimicrobial activity by a chemical synthesis method;
(B) translating the polypeptide with antimicrobial activity from the above-mentioned in-vitro translation template molecule;
(C) culturing the above-mentioned transformant; or
(D) culturing at least one type of lactic acid bacteria selected from the group consisting of accession numbers NITE BP-03198, NITE BP-03200, and NITE BP-03201.

A method of producing a combination of polypeptides with antimicrobial activity according to the present embodiment comprises one of:
(A) synthesizing individual polypeptides of the above-mentioned combination of polypeptides by a chemical synthesis method;
(B) translating the individual polypeptides of the combination of polypeptides from the above-mentioned in-vitro translation template molecule;
(C) culturing the above-mentioned transformant; or
(D) culturing lactic acid bacteria under an accession number of NITE BP-03200.

The polypeptide or the individual polypeptides of the combination of polypeptides according to the present embodiment can be produced by a widely-used protein chemical synthesis method. Examples of the protein chemical synthesis method include a liquid-phase method and a solid-phase method.

The polypeptide or the individual polypeptides of the combination of polypeptides according to the present embodiment can be produced with the use of a widely-used cell-free protein synthesis system, by translating the polypeptide from the in-vitro translation template molecule. Examples of the cell-free protein synthesis system include a wheat germ cell-free protein synthesis system.

The polypeptide or the individual polypeptides of the combination of polypeptides according to the present embodiment can be produced by culturing the above-mentioned transformant.

The culturing of the transformant may be carried out by the method for culturing the host cell used in the transformation. In the case where the transformant is a microorganism, the culturing can be carried out by using various types of media containing, as appropriate, carbon sources, nitrogen sources, organic and inorganic salts, and/or the like that are usually used for culturing the microorganism, for example. Herein, examples of the carbon sources include glucose, dextrin, soluble starch, sucrose, and the like. Examples of the nitrogen sources include inorganic substances such as ammonium salts and nitric acid salts as well as organic substances such as corn steep liquor, peptone, casein, meat extract, soybean dregs, and potato extract liquid. Examples of the inorganic matter include calcium chloride, sodium dihydrogen phosphate, magnesium chloride, and the like. To the medium, yeast extract, vitamins, growth-promoting factors, and/or the like may be further added.

As a result of culturing the transformant, a culture that contains the polypeptide according to the present embodiment is obtained. For example, the polypeptide according to the present embodiment may be accumulated inside the transformant and/or outside the transformant (for example, in the culture supernatant of the transformant). The polypeptide according to the present embodiment can be obtained from the transformant and/or from the culture supernatant thereof. The polypeptide according to the present embodiment may be a polypeptide obtained by disruption, lysis, extraction, and purification of the transformant as appropriate. The disruption, lysis, extraction, and the like can be carried out by known methods. Examples of the methods to do these operations include an ultrasonic disruption method, a dyno-mill method, beads disruption, french press disruption, and lysozyme treatment. One of these methods may be used alone, or two or more of these may be used in combination as appropriate. The purification can be carried out by a known method that is used for polypeptide purification. Examples of the method include ammonium sulfate fractionation, ion-exchange chromatography, hydrophobic chromatography, affinity chromatography, gel-filtration chromatography, and isoelectric precipitation. One of these methods may be used alone, or two or more of these may be used in combination as appropriate. The transformant may be collected from the culture by centrifugation and/or the like. In the case where the polypeptide according to the present invention is accumulated in the culture supernatant, the culture supernatant can be obtained by centrifugation and/or the like, and the polypeptide according to the present invention can be collected from the culture supernatant. The polypeptide according to the present embodiment may be a fraction of the culture supernatant of the transformant.

Alternatively, the polypeptide according to the present embodiment can be obtained by culturing at least one type of lactic acid bacteria selected from the group consisting of accession numbers NITE-BP-03198, NITE BP-03200, and NITE BP-03201 in a proper medium such as MRS liquid medium. The polypeptide according to the present embodiment may be collected, extracted, and purified from the culture of the above-mentioned lactic acid bacteria by the same method as used in the transformant culturing.

Examples of bacteria having the gene of the polypeptide according to the present embodiment include NITE-BP-03198, NITE-BP-03200, and NITE-BP-03201. NITE-BP-03198, NITE-BP-03200, and NITE-BP-03201 are bacteria internationally deposited in National Institute of Technology and Evaluation (NITE) Patent Microorganisms Depositary (NPMD, Address: Room 122, 2-5-8, Kazusakamatari, Kisarazu, Chiba, Japan 292-0818) under the Budapest Treaty as Accession Number NITE BP-03198 (Original date of deposit: April 9, 2020), Accession Number NITE BP-03200 (Original date of deposit: April 9, 2020), and Accession Number NITE BP-03201 (Original date of deposit: April 9, 2020), respectively. All of these bacteria belong to lactic acid bacteria named Lactiplantibacillus plantarum.

### [Examples]

In the following, a more detailed description will be given of the present invention by way of Examples, but these Examples are not intended to limit the scope of the present invention.

### [Experiment 1: Prediction of Antimicrobial Peptide by Genomic Analysis of Lactic Acid Bacteria Named Lactiplantibacillus Plantarum (NITE BP-03198, BP-03200, or NITE BP-03201)]

For each of three types of lactic acid bacteria named Lactiplantibacillus plantarum (NITE BP-03198, BP-03200, or NITE BP-03201), long-read sequence data was acquired with the use of PacBio RSII sequencer (Pacific Biosciences) and a short-read sequence was acquired with the use of NovaSeq6000 sequencer (Illumina). The short sequence data and the long sequence data thus acquired were subjected to hybrid assembly processing on Unicycler, and thereby the genome sequence of each of the three types of lactic acid bacteria was acquired. Subsequently, the genome sequence thus acquired was subjected to gene prediction on DFAST, followed by HMMER searching for homologous sequences in known antimicrobial peptides and antiSMASH searching for secondary metabolite-related genes, to acquire base sequences and amino acid sequences which were antimicrobial peptide candidates. Results are given in Table 1. In Table 1, descriptions (y=c) and (y=t) mean that the base denoted as y in the base sequence is cytosine and thymine, respectively. In Table 1, (X=A) and (X=V) mean that the amino acid residue denoted as X in the amino acid sequence is alanine and valine, respectively.

**[Table 1]**

| No. | Lactic acid bacteria | Gene ID /LOCUS | CDS Base sequence | Amino acid sequence of precursor polypeptide | |
|---|---|---|---|---|---|
| 1 | NITE BP-03198 | LOCUS 30660 | SEQ ID NO: 17 | SEQ ID NO: 25 | 45 residues |
| 2 | NITE BP-03198 | LOCUS 03600 | SEQ ID NO: 18 | SEQ ID NO: 26 | 49 residues |
| 3 | NITE BP-03200 | LOCUS 29660 | SEQ ID NO: 19 | SEQ ID NO: 27 | 55 residues |
| 4 | NITE BP-03200 | LOCUS 29680 | SEQ ID NO: 20 | SEQ ID NO: 28 | 58 residues |
| 5 | NITE BP-03200 | LOCUS _30290 | SEQ ID NO: 21 (y=c) | SEQ ID NO: 29 (X=A) | 50 residues |
| 6 | NITE BP-03200 | LOCUS 03310 | SEQ ID NO: 23 | SEQ ID NO: 31 | 57 residues |
| 7 | NITE BP-03200 | LOCUS 03320 | SEQ ID NO: 24 | SEQ ID NO: 32 | 59 residues |
| 8 | NITE BP-03201 | LOCUS_32200 | SEQ ID NO: 21 (y=t) | SEQ ID NO: 29 (X=V) | 50 residues |
| 9 | NITE BP-03201 | LOCUS 32840 | SEQ ID NO: 22 | SEQ ID NO: 30 | 62 residues |

### [Experiment 2: Homology Search Results (Amino Acid Sequences)]

In order to determine whether the antimicrobial peptides predicted by the genomic analysis were novel peptides, homology search was carried out with respect to the amino acid sequences of known peptides registered in BLAST (Query: Amino acid sequences free of leader peptide sequences) (Database: All non-redundant GenBank CDS translations+PDB+SwissProt+PIR+PRF excluding environmental samples from WGS projects). Results showed that many of the antimicrobial peptides predicted by the genomic analysis were similar to hypothetical protein, with homology of 65% to 100%. Each of Locus _29660, Locus_03310, and Locus_03320 was predicted to be bacteriosin based on the gene sequence, but no report was found regarding the peptide. Locus_32840 was similar to mutacin 1140, with only four different amino acid residues. From these results, it is conceivable that the peptides predicted by the genomic analysis were novel antimicrobial peptides.

### [Experiment 3: Isolation and Purification of Antimicrobial Substance]

### Purification of Active Component

Purification of an active component was performed by the following procedure. Each of the above-mentioned three types of lactic acid bacteria was inoculated in MRS medium and cultured under the conditions at 30°C for 24 hours, and thereby culture liquid was obtained. The culture liquid thus obtained was centrifuged at 6000 g for 10 minutes to give culture supernatant. Further, the culture supernatant liquid thus obtained was filtered through a cellulose acetate membrane with pore size of 0.22 µm for removing bacterial cells. To 500 mL of the lactic acid bacteria culture supernatant liquid, ammonium sulfate was added in a final concentration of 80%, and the resultant was stirred overnight at 6°C to precipitate protein containing an antimicrobial peptide. The protein thus precipitated was collected by 60 minutes of centrifugation at 13000 g, and then dissolved in 50 mL of 50-mM sodium phosphate buffer (pH 5.6; buffer A). Subsequently, the solution in which the protein was dissolved was fractionated with the use of AKTA pure Fraction Collecter (GE Healthcare) connected to Hitrap SP FF 1 mL (GE Healthcare). Regarding the mobile phase, buffer A was used as a mobile phase A, and 50-mM sodium phosphate buffer (pH 5.6) containing 1 M NaCl was used as a mobile phase B. The flow speed during fractionation was set at 1 mL/min. Gradient conditions were set as follows: the initial mobile phase composition was set at 0% mobile phase B, the final composition was set at 100% mobile phase B, and the gradient time was set at 10 minutes. Fractions were collected each in an amount of 1 mL.

Among all the fractions, an active fraction was further purified with the use of AKTA pure Fraction Collecter (GE Healthcare) connected to 1 mL RESOURCE PRC (GE Healthcare). Regarding the mobile phase, 0.1% formic acid aqueous solution was used as a mobile phase A, and 0.1% formic acid and ethanol were used as a mobile phase B. The flow speed during purification was set at 1 mL/min. Gradient conditions were set as follows: the initial mobile phase composition was set at 0% mobile phase B, the final composition was set at 100% mobile phase B, and the gradient time was set at 10 minutes. Fractions were collected each in an amount of 1 mL. The purified active fraction was stored at -30°C. The antimicrobial activity of the fraction thus obtained in each purification step was evaluated in the following manner.

### Antimicrobial Activity Test

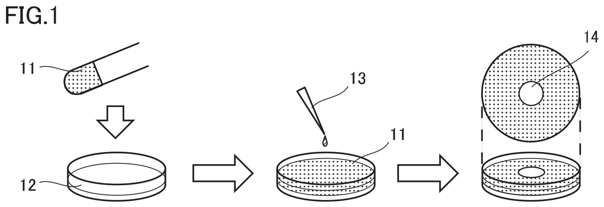

Antimicrobial activity of the antimicrobial peptide was evaluated by a spot-on-lawn method that involved spotting 10 µL of a test liquid 13 on the test bacteria (lawn) (on a soft agar medium 11) (Fig. 1). As the test bacteria, Lactobacillus sakei (NBRC15893) was used. The specific procedure is as follows. Firstly, the test bacteria that were cultured overnight were mixed and diluted with Lactobacilli AOAC agar (soft agar medium 11) in a density of 4×10⁷ CFU/mL, and was spread over an MRS plate containing 2% agar (an agar medium 12). Test liquid 13 was spotted thereon, and incubated overnight at 30°C, followed by evaluation of the size of a growth inhibition circle 14 (Fig. 1). It can be determined that the larger the growth inhibition circle, the higher the antimicrobial activity. The fraction with highest antimicrobial activity as determined in the antimicrobial activity test was regarded as a purified active fraction and used in Experiment 4 and Experiment 5 described below.

### [Experiment 4: Structure Determination of Antimicrobial Peptide, 1]

30 µl of an ethanol solution that was expected to contain an antimicrobial peptide of interest (a purified active fraction) was evaporated to dryness, and then dissolved in 50% methanol solution to make 10 µl in total. The purified active fraction thus dissolved was subjected to measurement by liquid chromatography-tandem mass spectrometry (LC/MS/MS) with electrospray ionization for interface. As the liquid chromatograph and autosampler, Ultimate3000 RSLCnano (Thermo Fisher Scientific) was used. An aqueous solution of 0.5% formic acid and 0.05% trifluoroacetic acid (TFA) was used as a mobile phase A, and an acetonitrile solution of 0.5% formic acid and 0.05% TFA was used as a mobile phase B. The flow speed during analysis was 400 nL/min. Gradient conditions were set as follows: the initial mobile phase composition was set at 5% mobile phase B, the final composition was set at 90% mobile phase B, and the gradient time was set at 40 minutes. As the column for analysis, NTCC-360/100-3-125 (Nikkyo technos) was used. As the mass spectrometer, orbitrap elite (Thermo Fisher Scientific) was used, and the MS spectrum for the full-length peptide was acquired in the full-scan mode. Separately, the mass (theoretical value) of the peptide calculated in Experiment 1 above was checked against the spectrum data (measured value) acquired by MS analysis, and when the presence was confirmed, its predicted sequence was written out. Results are given in the "Determination Method 1" section in Table 2.

### [Experiment 5: Structure Determination of Antimicrobial Substance, 2]

### Trypsin Digestion

A peptide of interest was digested by a conventional procedure. Specifically, 10 µl of a fraction with antimicrobial activity (a purified active fraction) was mixed with 10 µl of 2×sample buffer and separated by Tris-Tricin SDS Page (both from invitrogen), followed by cutting out of the band around a molecular weight of 3000. As the 2×sample buffer, "Novex Tricine SDS Sample Buffer" was used. As the SDS Page gel, "Novex 10 to 20%, Tricine, 1.0 mm, Mini Protein Gels 15 well" was used. The gel thus cut out was reduced and alkylated with the use of Iodoacetamide (Thermo Fisher Scientific), followed by trypsin digestion at 37°C for 16 hours. For digestion, Sequencing Grade Modified Trypsin (promega) was used. Subsequently, acetonitrile was used to shrink the gel to extract the digested peptide. The resulting extract was desalted and analyzed by MS. The conditions of the MS analysis were the same as in Experiment 4, with the gradient time changed to 30 minutes as well as data acquisition performed on the Data Dependent Acquisition (DDA) mode, and thereby an MS spectrum and an MS/MS spectrum were acquired.

### Mascot Search

The protein frame obtained by genomic analysis was converted into a FASTA file, and imported to MASCOT (Matrix Science) (the entire strain). The database was checked against the MS/MS spectrum data and MS spectrum data obtained by measurement (tolerance: precursor 30 ppm, fragment 0.8 Da, maximum charge 2+). Those that were highly reliable based on the score calculated on Proteome discoverer (Thermo Fisher Scientific), that satisfied requirements as a peptide (not longer than about 50 amino acid residues), and that were not nucleoproteins, for example, were selected as antimicrobial peptide candidates. Results are given in the "Determination Method 2" section in Table 2. "+" indicates that an antimicrobial peptide with a matching internal sequence was detected, and empty cells indicate that such a peptide was not detected. Even when it was not detected, this does not deny the possibility of the presence of such a peptide in the purified active fraction.

**[Table 2]**

| No. | Lactic acid bacteria | Gene ID/LOCUS | Amino acid sequence of mature polypeptide | Determination Method 1 | | Determination Method 2 |
|---|---|---|---|---|---|---|
| | | | | Measured value | Theoretical value | Matching internal sequence |
| 1 | NITE BP-03198 | LOCUS_30660 | MSKKQWYRLGERVGRVAVFIGGGLLFK (SEQ ID NO: 1) | 3110.7 | 3110.7(W)^{*} | + |
| 2 | NITE BP-03198 | LOCUS_03600 | KTKTISLMSGLQVPHAFTKLLKALGGHH (SEQ ID NO: 2) | 3012.7 | 3012.7 | + |
| 3 | NITE BP-03200 | LOCUS_29660 | WLQYLIPVVVSGLNHTDQIMRGWKKGYHRHH (SEQ ID NO: 3) | 3768.0 | 3768.0 | |
| 4 | NITE BP-03200 | LOCUS_29680 | SSSLLNTAWRKFGQIAGTVTSAGLDAGWIRAHKYGHGRSH (SEQ ID NO: 4) | 4293.2 | 4293.2 | + |
| 5 | NITE BP-03200 | LOCUS_30290 | SHAYNAAAIRRNNKSMKGAASFAKKFFPAFF (SEQ ID NO: 5) | 3447.8 | 3447.8 | + |
| 6 | NITE BP-03200 | LOCUS_03310 | SNNKFWTWAGYTYENWRISSRRAFNLRQRKNTMTHH (SEQ ID NO: 13) | 4556.2 | 4556.2 | |
| 7 | NITE BP-03200 | LOCUS_03320 | IWQWIVGGLGFLAGDAWSHSDQISSGIKKRKKKGYGY (SEQ ID NO: 14) | 4136.2 | 4136.2 | |
| 8 | NITE BP-03201 | LOCUS_32200 | SHAYNAAAIRRNNKSMKGAASFVKKFFPAFF (SEQ ID NO: 5) | 3475.8 | 3475.8 | |
| 9 | NITE BP-03201 | LOCUS_32840 | FKSWSLCTFGCGHTGSFNSFCC (SEQ ID NO: 6) | 2264.2 | 2264.2 | |

| | | | | | | |
|---|---|---|---|---|---|---|
| * indicates the mass when tryptophan in the sequence is in the oxidized form. | | | | | | |

### [Experiment 6: Antimicrobial Activity of Synthesized Peptide]

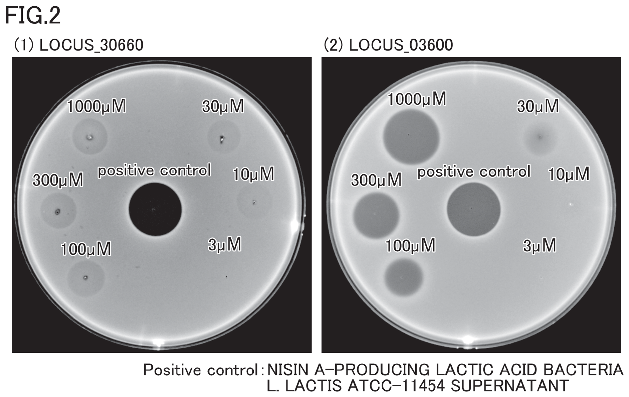

Chemically-synthesized peptides (full-length sequences and partial sequences) used in the present experiment were purchased from Hokkaido System Science, which were products synthesized by solid phase synthesis. For each of the chemically-synthesized peptides, an antimicrobial activity test was carried out in the same manner as in Experiment 3 and the lower limit to the concentration at which a growth inhibition circle was formed (the lowest concentration) was determined (Fig. 2). At this time, the test liquid was prepared as a solution in water having a desired concentration. Results are given in Table 3. In Table 3, the "Alone" section indicates the lowest concentration at which the antimicrobial peptide alone exhibited antimicrobial activity. The "Combination" section indicates the lowest concentration at which the two types of antimicrobial peptides combined in equimolar amounts exhibited antimicrobial activity (the lowest concentration for each of the two types of antimicrobial peptides).

The results in Table 3 indicate that the polypeptide as set forth in SEQ ID NO: 3 and the polypeptide as set forth in SEQ ID NO: 4 used in combination exhibit higher antimicrobial activity than when used alone. It is also indicated that the polypeptide as set forth in SEQ ID NO: 13 and the polypeptide as set forth in SEQ ID NO: 14 used in combination exhibit higher antimicrobial activity than when used alone.

**[Table 3]**

| No. | Lactic acid bacteria | Gene ID/LOCUS | SEQ ID NO of amino acid sequence | Antimicrobial activity* (Lowest concentration) | |
|---|---|---|---|---|---|
| | | | | Alone | Combination |
| 1 | NITE BP-03198 | LOCUS 30660 | SEQ ID NO: 1 | 10 µM | ND** |
| 2 | NITE BP-03198 | LOCUS 03600 | SEQ ID NO: 2 | 30 µM | ND |
| 3 | NITE BP-03200 | LOCUS 29660 | SEQ ID NO: 3 | 10 µM | 0.15 µM |
| 4 | NITE BP-03200 | LOCUS 29680 | SEQ ID NO: 4 | 3 µM | |
| 5 | NITE BP-03200 | LOCUS 30290 | SEQ ID NO: 5 (X=A) | 100 µM | ND |
| 6 | NITE BP-03200 | LOCUS 03310 | SEQ ID NO: 13 | >1000 µM | 1.5 µM |
| 7 | NITE BP-03200 | LOCUS 03320 | SEQ ID NO: 14 | >1000 µM | |
| 8 | NITE BP-03201 | LOCUS32200 | SEQ ID NO: 5 (X=V) | 30 µM | ND |

| | | | | | |
|---|---|---|---|---|---|
| * The antimicrobial activity refers to antimicrobial activity on L. Sakei. ** Not Determined | | | | | |

### [Experiment 7: Wide pH-Range Stability Test]

For the polypeptides as set forth in SEQ ID NOs: 1 to 5, 13, and 14 in Table 3, evaluation was carried out to see if they had antimicrobial activity over a wide range of pH. As a Comparative Example, a commercially available nisin A (manufactured by Sigma-Aldrich) was used. Firstly, each peptide was dissolved in the following buffer to prepare a solution of a concentration ten times the lowest concentration specified in Table 3: sodium phosphate buffer (50 mM, pH4), 1x phosphate buffer saline (pH7.4), ammonium chloride-ammonia buffer (50 mM, pH10.0). The test solutions thus prepared at respective pHs were incubated at 27°C for 24 hours. Then, with the use of Lactobacillus sakei (NBRC15893) strain as test bacteria and in the same manner as in Experiment 3, an antimicrobial activity test was carried out to measure the antimicrobial activity of the test solutions incubated at respective pHs. As a control, a test solution prepared by diluting the peptide of interest in water was used. Antimicrobial activity was evaluated based on the size of the spot (the size of the growth inhibition circle). When the size of the spot was equivalent to that of the control (when the diameter of the spot was 80% or more of the control), it was rated as A, and when the size was relatively small but antimicrobial activity was still detected, it was rated as B. When the growth inhibition circle was not observed, it was rated as C. It can be determined that the peptides rated as A or B are stable at the test pHs. Results are given in Table 4 (in the "pH Stability" section). In the "SEQ ID NO or substance name of peptide" section in Table 4, each of "SEQ ID NO: 3 & SEQ ID NO: 4" and "SEQ ID NO: 13 & SEQ ID NO: 14" means that a combination of the two types of peptides was used in equimolar amounts. The results in Table 4 indicate that the polypeptides as set forth in SEQ ID NOs: 1 to 5, 13, and 14 are stable and have antimicrobial activity over a wide range of pH.

### [Experiment 8: Thermal Stability Test]

For the polypeptides as set forth in SEQ ID NOs: 1 to 5, 13, and 14 in Table 3, evaluation was carried out to see if they had antimicrobial activity after heat treatment. As a Comparative Example, a commercially available nisin A (manufactured by Sigma-Aldrich) was used. Firstly, each peptide was dissolved in water to prepare a solution of a concentration ten times the lowest concentration specified in Table 3. The test solution thus prepared was subjected to heat treatment at 80°C for 30 minutes, or at 100°C for 30 minutes, or at 121°C for 15 minutes. The heat treatment at 121°C was carried out with the use of an autoclave "LSX-700" manufactured by TOMY Seiko. Then, with the use of Lactobacillus sakei (NBRC15893) strain as test bacteria and in the same manner as in Experiment 3, an antimicrobial activity test was carried out to measure the antimicrobial activity of the test solutions after heat treatment. As a control, a sample solution with no heat treatment was used. Antimicrobial activity was evaluated based on the size of the spot (the size of the growth inhibition circle); when the size of the spot was equivalent to that of the control (when the diameter of the spot was 80% or more of the control), it was rated as A, and when the size was relatively small but activity was still detected, it was rated as B. When the spot was not observed, it was rated as C. It can be determined that the peptides rated as A or B are stable at the test temperatures. Results are given in Table 4 (in the "Thermostability" section). In the "SEQ ID NO or substance name of peptide" section in Table 4, each of "SEQ ID NO: 3 & SEQ ID NO: 4" and "SEQ ID NO: 13 & SEQ ID NO: 14" means that a combination of the two types of peptides was used in equimolar amounts. The results in Table 4 indicate that the polypeptides as set forth in SEQ ID NOs: 1 to 5, 13, and 14 have antimicrobial activity at temperatures within the range of 80°C to 121°C.

**[Table 4]**

| Origin | SEQ ID NO or substance name of peptide | Number of amino acid residues | Structure | pH Stability | | | Thermostability | | |
|---|---|---|---|---|---|---|---|---|---|
| | | | | pH4 | pH7 | pH10 | 80°C | 100°C | 121°C |
| NITE BP-03198 | SEQ ID NO: 1 | 27 | Linear | A | A | A | A | A | A |
| NITE BP-03198 | SEQ ID NO: 2 | 28 | Linear | A | A | A | A | A | A |
| NITE BP-03200 | SEQ ID NO: 3 | 31 | Linear | A | A | A | A | A | A |
| NITE BP-03200 | SEQ ID NO: 4 | 40 | Linear | A | A | B | A | A | A |
| NITE BP-03200 | SEQ ID NO: 5 (X=A) | 31 | Linear | A | A | B | A | A | A |
| NITE BP-03200 | SEQ ID NO: 3 & SEQ ID NO: 4 | 31/40 | Linear Pair | A | A | A | A | A | B |
| NITE BP-03200 | SEQ ID NO: 13 & SEQ ID NO: 14 | 36/37 | Linear Pair | A | B | B | A | A | A |
| NITE BP-03201 | SEQ ID NO: 5 (X=V) | 31 | Linear | A | A | A | A | A | A |
| Commercially available product | Nisin A | 34 | Intramolecular crosslinking | A | C | C | C | C | C |

| | | | | | | | | | |
|---|---|---|---|---|---|---|---|---|---|
| A: Good activity, B: Slight influence on activity, C: No activity | | | | | | | | | |

### [Experiment 9: Antimicrobial Activity Test on Novel Antimicrobial Peptide]

For the polypeptides as set forth in SEQ ID NOs: 1 to 5, 13, and 14 in Table 3, a test was carried out to see what type of microorganisms they exhibit antimicrobial activity against. As a Comparative Example, a commercially available nisin A (manufactured by Sigma-Aldrich) was used. Firstly, each peptide was dissolved in MRS medium to prepare a solution of a concentration of 20 µM to 1000 µM. From the resultant, 10 µl was taken and mixed with various test bacteria and 90 µl of medium intended for culturing the test bacteria, followed by culturing for 24 hours to 48 hours. After culturing, OD₆₃₀ was measured to check the number of bacteria. When OD₆₃₀ increased or turbidity was visually observed as compared to the culture liquid before culturing, it can be determined that the test bacteria had grown. Results are given in Table 5. When the test bacteria had not grown, it was determined that antimicrobial activity was exhibited and "+" was entered in Table 5. In the "SEQ ID NO or substance name of peptide" section in Table 5, each of "SEQ ID NO: 3 & SEQ ID NO: 4" and "SEQ ID NO: 13 & SEQ ID NO: 14" means that a combination of the two types of peptides were used in equimolar amounts. The results in Table 5 indicate that the polypeptides as set forth in SEQ ID NOs: 1 to 5, 13, and 14 were effective against gram-positive bacteria and some of these polypeptides were effective against gram-negative bacteria such as E. coli and fungi such as C. albicans.

**[Table 5]**

| Origin | SEQ ID NO or substance name of peptide | Test conc. | Test bacteria | | | | |
|---|---|---|---|---|---|---|---|
| | | | *Lactobacillus sakei* | *Streptococcus uberis* | *Candida albicans* | *Escherichia coli* | *Staphylococcus aureus* |
| NITE BP-03198 | SEQ ID NO: 1 | 30 µM | + | + | + | + | + |
| NITE BP-03198 | SEQ ID NO: 2 | 30 µM | + | + | - | - | - |
| NITE BP-03200 | SEQ ID NO: 13 & SEQ ID NO: 14 | 2 µM | + | + | - | - | + |
| NITE BP-03200 | SEQ ID NO: 3 | 20 µM | + | + | - | - | - |
| NITE BP-03200 | SEQ ID NO: 4 | 20 µM | + | + | - | - | + |
| NITE BP-03200 | SEQ ID NO: 3 & SEQ ID NO: 4 | 10 µM | + | + | + | - | + |
| NITE BP-03201 | SEQ ID NO: 5 (X=V) | 20 µM | + | + | - | - | - |
| Commercially available product | Nisin A | 25 µM | + | + | - | - | + |

| | | | | | | | |
|---|---|---|---|---|---|---|---|
| +: Antimicrobial activity observed, -: No antimicrobial activity observed | | | | | | | |

## Claims

1. A polypeptide with antimicrobial activity, comprising:
an amino acid sequence with a sequence identity of 85% to 100% to an amino acid sequence as set forth in any one of SEQ ID NO: 1 to SEQ ID NO: 6; or
an amino acid sequence as set forth in any one of SEQ ID NO: 1 to SEQ ID NO: 6 with deletion, substitution, insertion, and/or addition of one or several amino acid residues.

2. A nucleic acid comprising a base sequence encoding the polypeptide with antimicrobial activity according to claim 1.

3. The nucleic acid according to claim 2, comprising a base sequence as set forth in any one of SEQ ID NO: 7 to SEQ ID NO: 12.

4. A vector comprising the nucleic acid according to claim 2.

5. An in-vitro translation template molecule including the nucleic acid according to claim 2 or 3.

6. The in-vitro translation template molecule according to claim 5, wherein the in-vitro translation template molecule is an RNA.

7. A transformant comprising the nucleic acid according to claim 2 or 3 or the vector according to claim 4.

8. A method of producing a polypeptide with antimicrobial activity, the method comprising one of:
(A) synthesizing the polypeptide with antimicrobial activity according to claim 1 by a chemical synthesis method;
(B) translating the polypeptide with antimicrobial activity from the in-vitro translation template molecule according to claim 5;
(C) culturing the transformant according to claim 7; and
(D) culturing at least one type of lactic acid bacteria selected from the group consisting of accession numbers NITE BP-03198, NITE BP-03200, and NITE BP-03201.

9. A combination of polypeptides with antimicrobial activity, comprising:
a combination of a first polypeptide and a second polypeptide; or
a combination of a third polypeptide and a fourth polypeptide, wherein
the first polypeptide includes an amino acid sequence with a sequence identity of 85% to 100% to an amino acid sequence as set forth in SEQ ID NO: 3, or the amino acid sequence as set forth in SEQ ID NO: 3 with deletion, substitution, insertion, and/or addition of one or several amino acid residues,
the second polypeptide includes an amino acid sequence with a sequence identity of 85% to 100% to an amino acid sequence as set forth in SEQ ID NO: 4, or the amino acid sequence as set forth in SEQ ID NO: 4 with deletion, substitution, insertion, and/or addition of one or several amino acid residues,
the third polypeptide includes an amino acid sequence with a sequence identity of 85% to 100% to an amino acid sequence as set forth in SEQ ID NO: 13, or the amino acid sequence as set forth in SEQ ID NO: 13 with deletion, substitution, insertion, and/or addition of one or several amino acid residues, and
the fourth polypeptide includes an amino acid sequence with a sequence identity of 85% to 100% to an amino acid sequence as set forth in SEQ ID NO: 14, or the amino acid sequence as set forth in SEQ ID NO: 14 with deletion, substitution, insertion, and/or addition of one or several amino acid residues.

10. A combination of nucleic acids including base sequences respectively encoding individual polypeptides of the combination of polypeptides with antimicrobial activity according to claim 9, wherein
the combination of nucleic acids includes a combination of a first nucleic acid and a second nucleic acid or a combination of a third nucleic acid and a fourth nucleic acid,
the first nucleic acid includes a base sequence encoding the first polypeptide,
the second nucleic acid includes a base sequence encoding the second polypeptide,
the third nucleic acid includes a base sequence encoding the third polypeptide, and
the fourth nucleic acid includes a base sequence encoding the fourth polypeptide.

11. The combination of nucleic acids according to claim 10, wherein
the first nucleic acid includes a base sequence as set forth in SEQ ID NO: 9,
the second nucleic acid includes a base sequence as set forth in SEQ ID NO: 10,
the third nucleic acid includes a base sequence as set forth in SEQ ID NO: 15, and
the fourth nucleic acid includes a base sequence as set forth in SEQ ID NO: 16.

12. A vector comprising the combination of nucleic acids according to claim 10.

13. An in-vitro translation template molecule including the combination of nucleic acids according to claim 10 or 11.

14. The in-vitro translation template molecule according to claim 13, wherein the in-vitro translation template molecule is an RNA.

15. A transformant comprising the combination of nucleic acids according to claim 10 or 11 or the vector according to claim 12.

16. A method of producing a combination of polypeptides with antimicrobial activity, the method comprising one of:
(A) synthesizing individual polypeptides of the combination of polypeptides according to claim 9 by a chemical synthesis method;
(B) translating the individual polypeptides of the combination of polypeptides from the in-vitro translation template molecule according to claim 13;
(C) culturing the transformant according to claim 15; and
(D) culturing lactic acid bacteria under an accession number of NITE BP-03200.

17. An antimicrobial composition comprising:
the polypeptide with antimicrobial activity according to claim 1 or the combination of polypeptides with antimicrobial activity according to claim 9; and
an additive.
